# EUROPÄISCHE PATENTSCHRIFT

(11) **EP 1 896 584 B1**
(45) Veröffentlichungstag und Bekanntmachung des Hinweises auf die Patenterteilung: **10.02.2010**
(21) Anmeldenummer: 06762108.6
(22) Anmeldetag: 21.06.2006
(51) Int. Cl.: C12N 15/10

(54) **SELEKTION VON PHOSPHATASE KODIERENDEN NUKLEINSÄUREMOLEKÜLEN**
SELECTION OF PHOSPHATASE-CODING NUCLEIC ACID MOLECULES
SÉLECTION DE MOLÉCULES D'ACIDES NUCLÉIQUES CODANT DES PHOSPHATASES

(30) Priorität: 22.06.2005 DE 102005030552
(43) Veröffentlichungstag der Anmeldung: 12.03.2008
(73) Patentinhaber: Eucodis Bioscience GmbH, 1235 Wien (AT)
(72) Erfinder: STROBEL, Heike, 1230 Wien (AT)
(74) Vertreter: Schwahn, Hartmut
(86) Internationale Anmeldenummer: PCT/EP2006/005936
(87) Internationale Veröffentlichungsnummer: WO 2006/136383

(56) Entgegenhaltungen:
- EP-A- 0 100 561
- US-A1- 2004 005 695
- YANG KECHAO ET AL: "A new activity for an old enzyme: Escherichia coli bacterial alkaline phosphatase is a phosphite-dependent hydrogenase." PROCEEDINGS OF THE NATIONAL ACADEMY OF SCIENCES OF THE UNITED STATES OF AMERICA. 25 MAY 2004, Bd. 101, Nr. 21, 25. Mai 2004 (2004-05-25), Seiten 7919-7924, XP002396919 ISSN: 0027-8424
- MIKSCH G ET AL: "Overexpression of the phytase from Escherichia coli and its extracellular production in bioreactors." APPLIED MICROBIOLOGY AND BIOTECHNOLOGY. SEP 2002, Bd. 59, Nr. 6, September 2002 (2002-09), Seiten 685-694, XP002396920 ISSN: 0175-7598
- MITCHELL DB ET AL: "THE PHYTASE SUBFAMILY OF HISTIDINE ACID PHOSPHATASES: ISOLATION OF GENES FOR TWO NOVEL PHYTASES FROM THE FUNGI ASPERGILLUS TERREUS AND MYCELIOPHTHORA THERMOPHILA" MICROBIOLOGY, SOCIETY FOR GENERAL MICROBIOLOGY, READING, GB, Bd. 143, 1997, Seiten 245-252, XP002097277 ISSN: 1350-0872
- GOLOVAN S ET AL: "CHARACTERIZATION AND OVERPRODUCTION OF THE ESCHERICHIA COLI APPA ENCODED BIFUNCTIONAL ENZYME THAT INHIBITS BOTH PHYTASE AND ACID PHOSPHATASE ACTIVITIES" CANADIAN JOURNAL OF MICROBIOLOGY, OTTAWA, CA, Bd. 46, Nr. 1, Januar 2000 (2000-01), Seiten 59-71, XP009000251 ISSN: 0008-4166
- MIKSCH G ET AL: "THE KIL GENE OF THE COLE1 PLASMID OF ESCHERICHIA COLI CONTROLLED BYA GROWTH-PHASE-DEPENDENT PROMOTER MEDIATES THE SECRETION OF A HETEROGOLOUS PERIPLASMIC PROTEIN DURING THE STATIONARY PHASE" ARCHIVES OF MICROBIOLOGY, BERLIN, DE, Bd. 167, Nr. 2-3, 1997, Seiten 143-150, XP001008219 ISSN: 0302-8933

### SEQUENCE LISTING

<110> CSO Eucodis GmbH
<120> Selektion von Phosphatase-Aktivität
<130> 19378
<160> 9
<170> PatentIn version 3.3
<210> 1
   <211> 33
   <212> DNA
   <213> Artificial
<220>
   <223> Primer
<400> 1
   catgccatgg catgccattg cagggtggcc tgt 33
<210> 2
   <211> 33
   <212> DNA
   <213> Artificial
<220>
   <223> Primer
<400> 2
   cccaagcttg tgaatgcttt tgcttgatct cag 33
<210> 3
   <211> 32
   <212> DNA
   <213> Artificial
<220>
   <223> Primer
<400> 3
   ccgctcgagc gaaagcgatc ttaatcccat tt 32
<210> 4
   <211> 33
   <212> DNA
   <213> Artificial
<220>
   <223> Primer
<400> 4
   gaagatcttc ccaaactgca cgccggtatg cgt 33
<210> 5
   <211> 33
   <212> DNA
   <213> Artificial
<220>
   <223> Primer
<400> 5
   ccgctcgagc gaacaaaacg ctaatcgccg cag 33
<210> 6
   <211> 33
   <212> DNA
   <213> Artificial
<220>
   <223> Primer
<400> 6
   gaagatcttc ctttcaccgc ttcattcaac acg 33
<210> 7
   <211> 35
   <212> DNA
   <213> Artificial
<220>
   <223> primer
<400> 7
   ccgctcgagc gaagcataag ctgtctgatc cttat 35
<210> 8
   <211> 33
   <212> DNA
   <213> Artificial
<220>
   <223> primer
<400> 8
   ggaagatctt tattttccgc ttctgtcggt cag 33
<210> 9
   <211> 33
   <212> DNA
   <213> Artificial
<220>
   <223> primer
<400> 9
   ggaagatctt tttccgcttc tgtcggtcag ttt 33

## Beschreibung

Die vorliegende Erfindung betrifft Verfahren zur Selektion von Nukleinsäuremolekülen kodierend für Proteine mit Phosphatase-Aktivität aus einer Bank zu untersuchender Nukleinsäuremoleküle.

Phosphatasen sind Proteine mit Phosphatase-Aktivität. Ein Protein ist eine Phosphatase beziehungsweise besitzt Phosphatase-Aktivität, wenn es in der Lage ist, von phosphathaltigen Substraten anorganisches Phosphat abzuspalten. Bekannte und wirtschaftlich interessante Phosphatasen sind beispielsweise die pflanzlichen Phytasen.

Bis zu 80 % des Phosphors in Nahrungsmitteln pflanzlichen Ursprungs kann durch Tiere, besonders monogastrische Tiere wie Schweine und Geflügel, metabolisch nicht verwertet werden. Infolgedessen gelangen Millionen Tonnen an Phosphor in die Umwelt und verursachen große Probleme, indem sie beispielsweise zu Algenblüte, Sauerstoffverminderung und zur Zerstörung von Nahrungsketten führen. Auf der anderen Seite ist es teilweise schwierig, mit herkömmlichen Futtermitteln, den Phosphorbedarf dieser Tiere zu decken. Es besteht daher der Bedarf, Futtermittel einzusetzen, deren Phosphorgehalt weitgehend metabolisiert werden kann.

Eine Möglichkeit, den Anteil metabolisierbaren Phosphors in Futtermitteln zu erhöhen, besteht darin, den Futtermitteln Phytase zuzusetzen. Phytasen sind Enzyme, welche die in pflanzlicher Nahrung enthaltene phosphorhaltige Komponente Phytinsäure beziehungsweise Phytat (Salz der Phytinsäure) hydrolysieren, wobei anorganisches Phosphat freigesetzt wird. Phytinsäure/Phytat ist ein *myo-*Inositol-1,2,3,4,5,6-Hexakis-Dihydrogen-Phosphat; es enthält ungefähr 14 bis 18 % Phosphor und in der Regel 12 bis 20 % Kalzium.

Phytat/Phytinsäure liegt in der Regel als stark negativ geladenes Molekül vor. Dadurch wird der Durchtritt durch biologische Membranen erschwert. Um Phytat/Phytinsäure bildet sich eine Hydrathülle, was dem Durchtritt des Moleküls durch eine biologische Membran beziehungsweise durch Poren ebenfalls entgegenwirkt. Beispielsweise kann das hydrierte Phytat-Molekül nicht die periplasmatische Membran von gram-negativen Bakterien durchdringen.

Phytase ist das einzig bekannte Enzym, das in der Lage ist, die aufeinander folgende Phosphathydrolyse an Kohlenstoffatom 1, 3 oder 6 im Inositolring des Phytats zu initiieren. 6-Phytasen (z.B. AppA) katalysieren die Phosphathydrolyse beginnend am Kohlenstoffatom Nr. 6, 3-Phytasen (Glucose-1-Phosphatase; setzt nur eine ortho-Phosphatgruppe frei) entsprechend an Kohlenstoffatom Nr. 3. Die Abspaltung der Phosphatgruppe durch Phytase führt zur Freisetzung von zweiwertigen Metallionen wie Kalzium, Eisen, Zink und anderen.

Phytasen kommen in einer Vielzahl verschiedener Mikroorganismen und Pflanzen sowie in Geweben einiger Tiere vor. Phytasen aus gram-negativen Bakterien sind intrazelluläre Proteine, die meistens periplasmatisch lokalisiert sind. Phytasen aus gram-positiven Bakterien und Pilzen sind hingegen extrazelluläre Proteine. Alle bekannten Phytasen sind monomere Proteine mit Ausnahme von Phytase B (PhyB) aus *Aspergillus niger*; PhyB ist ein Tetramer. Die Molekülmassen dieser Enzyme sind sehr variabel und betragen in der Regel zwischen 38 und 100 kDa. Phytasen aus Pilzen oder Hefen haben aufgrund der Glycosylierung ein höheres Molekulargewicht. Die Glycosylierung hat jedoch keinen Effekt auf die spezifische Aktivität und die thermische Stabilität der Phytase.

Die bekannten Phytasen sind weder strukturell sehr ähnlich, noch spalten sie anorganisches Phosphat über denselben Mechanismus. Phytasen lassen sich aufgrund der pH-Abhängigkeit ihrer Enzymaktivität in drei Hauptgruppen, nämlich saure, neutrale und alkalische Phosphatasen, einteilen. Für die Unterstützung des Phosphatmetabolismus in Futtermitteln für monogastrische Tiere kommen in erster Linie Phytasen aus der Gruppe der sauren Phosphatasen in Frage.

Die phylogenetische Analyse der Aminosäuresequenzen verschiedener Phytasen ergibt ebenfalls drei Hauptklassen. Diese Einteilung stimmt gut mit der Einteilung der Phytasen nach biochemischen oder katalytischen Eigenschaften überein:
Klasse I - Histidin saure Phosphatase-Phytase (HAP)
   Eine der Hauptklasse ist die Familie der HAP. Ihre Mitglieder sind unter anderem PhyA und PhyB aus *Aspergillus niger* und AppA aus *Escherichia coli.* Diese haben eine breite Substratspezifität. Sie sind in der Lage, Metall-freies Phytat im sauren pH-Bereich zu hydrolysieren. Ihnen gemeinsam ist das hochkonservierte Motiv RHGXRXP.
   Die Klasse kann weiter in drei Untergruppen unterteilt werden: PhyA, PhyB und PhyC, und zwar abhängig von der Homologie der Aminosäuresequenz und biochemischen Eigenschaften wie pH-Optimum oder der Stellungsspezifität der Phytathydrolyse.
Klasse II - β-Propeller-Phytasen (BPP) oder alkalische Phytasen
   Dazu zählen beispielsweise PhyC aus *Bacillus subtilis* und PhyA aus *Bacillus amyloliquefaciens* FZB45. Bacillus Phytase (β-Propeller) katalysiert die Hydrolyse der Phytinsäure in Inositol und ortho-Phosphat in Gegenwart zweiwertiger Elemente wie Kalzium, Eisen oder Zink. Diese Klasse unterscheidet sich von der HAP-Klasse in einer Reihe von Aspekten wie pH-Optimum, Molekülmasse, Tertiärstruktur, Substratspezifität und dem Erfordemis der Gegenwart von Kalzium-Ionen bei der Katalyse. Alkalische Phytasen aus *Bacillus* oder Pflanzensamen können aufgrund dieser biochemischen Unterschiede und aufgrund der phylogenetischen Daten als die weitere Gruppe PhyD klassifiziert werden.
Klasse III - Purple acid-Phosphatasen (PAP):
   Zu dieser Klasse gehört beispielsweise GmPhy aus der Sojabohne, *Glycine max* L. GmPhy wurde kürzlich aus den Cotyledonen keimender Sojabohnen isoliert. Der aktive Abschnitt von GmPhy besitzt das Motiv einer Purple acid-Phosphatase (PAP). Das geschätzte Molekulargewicht beträgt 70 bis 72 kDa, ähnlich wie andere pflanzliche PAP. Jedoch ist GmPhy die einzige bekannte PAP mit signifikanter Phytase-Aktivität. Der Vergleich der Aminosäuresequenz ergab eine 33 %ige Homologie zwischen GmPhy und PAP aus Kidneybohnen.

Indem die Phytasen aus der unverdaubaren Phytinsäure anorganisches Phosphat freisetzen, kann Phosphat aus dem Futtermittel absorbiert werden. Dadurch wird nicht nur der Phosphatabfall reduziert. Vielmehr besteht keine Notwendigkeit mehr, solche Futtermittel anderweitig mit metabolisierbarem Phosphat zu supplementieren.

Der Abbau von Phytinsäure beziehungsweise Phytat in Futtermitteln dient jedoch nicht allein zur Bereitstellung von metabolisierbarem Phosphat. Phytinsäure beziehungsweise Phytat, wie es in den Randschichten des Getreidekoms vorkommt, gilt als Komplexbildner für Metallionen und vermindert somit außerdem die Resorption von wichtigen Metallionen aus den Futtermitteln wie Kalzium, Eisen, Magnesium, Phosphor oder Zink. Bei Menschen wird der Zusammenhang zwischen einem durch bestimmte Vollkomprodukte induzierten Mineralstoffmangel und dem Vorkommen von Phytinsäure beziehungsweise Phytat in diesen Vollkornprodukten diskutiert. Es ist daher wünschenswert, den Anteil an Phytinsäure beziehungsweise Phytat in Vollkomprodukten zu reduzieren. Dies kann durch entsprechenden Einsatz von Phytase bei der Herstellung der Vollkornprodukte oder aber durch Zugabe von Phytase zu den Vollkomprodukten geschehen.

Auf der anderen Seite ist die Verwendung bekannter Phytasen in Futtermitteln durch die hohen Kosten bei der Bereitstellung der Phytasen begrenzt. Die Anwendbarkeit bekannter Phytasen in Futtermitteln wird auch dadurch limitiert, dass diese bei den hohen Temperaturen, die für die Pelletierung der Futtermittel verwendet werden (in der Regel mehr als 80°C), inaktiviert werden. Weiter verlieren bekannte Phytasen einen Großteil ihrer Aktivität während der Lagerung der Futtermittel. Eine Vielzahl der bekannten Phytasen ist schon deswegen ungeeignet, da sie nur in einem engen pH-Bereich, der im Verdauungstrakt in der Regel nicht vorliegt, stabil und/oder aktiv sind. Eine Reihe der bekannten Phytasen ist außerdem nicht gegen Abbau durch im Verdauungstrakt vorkommende Proteasen resistent. Darüber hinaus liegt die Phosphorquelle Phytinsäure beziehungsweise Phytat im Verdauungstrakt dieser Tiere hauptsächlich als Kalzium-Phytatkomplex vor, besonders dann, wenn das Futtermittel einen hohen Kalziumgehalt aufweist (cirka 3 bis 40 g/kg). Dieser Kalzium-Phytatkomplex kann aber nicht als Substrat für eine Vielzahl bekannter Phytasen dienen.

Es besteht daher Bedarf an neuen Phosphatasen, insbesondere Phytasen, die verbesserte biochemische und katalytische Eigenschaften aufweisen, wodurch sie für die Verwendung in der Futtermittel- und Nahrungsmittelindustrie besser geeignet sind. Proteine mit Phytase-Aktivität werden in einer Vielzahl verschiedener Organismen exprimiert. Nicht alle in der Natur vorkommenden Phytasen sind bisher charakterisiert oder isoliert worden; nicht alle Gene, die für Proteine mit Phytase-Aktivität kodieren, sind bekannt. Es besteht daher Bedarf, Selektionsverfahren bereitzustellen, mit deren Hilfe Gene, die eine bestimmte ausgewählte Phosphatase-Aktivität, besonders eine Phytase-Aktivität, kodieren, in besonders effektiver Weise detektiert und selektiert werden können.

Bekannte Selektionsverfahren, die das Ziel verfolgen, neue Gene aufzufinden, die eine bestimmte Funktion, beispielsweise eine Enzymaktivität kodieren, basieren in der Regel auf flüssigen Ansätzen in der wässrigen Phase, so genannten "liquid assays". Assays für die Selektion von Phosphatase, insbesondere Phytase kodierenden Genen basieren beispielsweise auf der Bildung eines Phosphormolybdat-Komplexes und der damit verbundenen Farbreaktion. Hoenig et al. (J. Biochem. Biophys. Methods, 1989, 19(2-3):249-251) beschreiben ein Assay basierend auf Malachitgrün; Greiner et al. (Arch. Biochem. Biophys. 1997, 341(2):201-206) beschreiben die Bestimmung der Phytaseaktivität mit einer Reagenzienlösung, enthaltend Heptamolybdat/Ammoniumvanadat; nach Heinonen und Lathi (Anal. Biochem. 1981, 113(2):313-317) kann Phytaseaktivität durch Reduktion des gebildeten Phosphormolybdat-Komplexes nachgewiesen werden. Diese bekannten Nachweisverfahren mögen für eine geringe Anzahl zu untersuchender Proben ausreichend sein. Soll jedoch eine komplette Bibliothek untersucht werden, die den Einsatz mehrerer Millionen verschiedener Klone erforderlich macht, sind die bekannten Assays ungeeignet. Diese wären bei der großen Anzahl verschiedener Klone extrem zeitraubend oder gar nicht kontrolliert durchfüh r-bar. Es besteht daher auch Bedarf an einem Selektionsverfahren, das auch mit einer großen Anzahl zu untersuchender Proben durchgeführt werden kann und besonders auch für eine automatisierte Anwendung einsetzbar ist. Es sollte sich dabei also um ein schnelles und einfaches Selektions-Assay handeln.

Yang und Metcalf (PNAS (2004) 101(21):7919-7924) schreiben delta-phoA-Mutanten für alkalische Phosphatase von *E. coli.* Die Phosphatase-Aktivität der Mutanten wurde mittels *p-*Nitrophenylphosphat (pNPP) in einer Farbreaktion nachgewiesen. Dazu wurden die Zellen in einem Phosphat-Mangelmedium gezüchtet.

Miksch et al. (Appl. Microbiol. Biotechnol. 2002, 59:685-694) beschreiben die Überexpression von Phytase in *E. coli.* Die intrazellulär exprimierte Phytase wird durch ein Transportsystem, welches auf dem *kil*-Gen des Phagen Lambda basiert, aus der *E. coli* Zelle ins Extrazellulärmedium ausgeschleust und dort im Assay nachgewiesen. In der verwandten US 2004/0005695 A1 wird ebenfalls das Kil-Protein zum Ausschleusen von rekombinanten Proteinen oder Enzymen aus *E. coli* beschrieben.

Davon ausgehend besteht das der vorliegenden Erfindung zugrunde liegende technische Problem im Wesentlichen darin, ein Verfahren zur Selektion eines Nukleinsäuremoleküls, das für eine ausgewählte Phosphatase-Aktivität kodiert, bereitzustellen. Besonders soll das Verfahren einfach mit an sich bekannten Mitteln durchzuführen sein und eine besonders gute und schnelle Selektion, auch bei einer großen Anzahl an zu untersuchenden Proben, ermöglichen.

Das zugrunde liegende technische Problem wird gelöst durch ein Verfahren zur Selektion eines Nukleinsäuremoleküls, das für ein Protein mit einer ausgewählten Phosphatase-Aktivität kodiert, wobei das Nukleinsäuremolekül aus einer Gruppe zu untersuchender Nukleinsäuremoleküle selektiert wird. Vorzugsweise ist die Gruppe eine Bank oder Bibliothek. Das erfindungsgemäße Verfahren enthält folgende Schritte, insbesondere besteht es daraus:
a) das zu untersuchende Nukleinsäuremolekül wird bereitgestellt;
b) in das Genom einer Wirtszelle, die eine Phosphatase-Defizienz zeigt beziehungsweise worin keine Phosphatase-Aktivität exprimiert wird, wird das zu untersuchende Nukleinsäuremolekül, und zusätzlich ein Nukleinsäuremolekül kodierend für das Kil-Protein es Phagen Lambda (λ), eingeführt;
c) die Wirtszelle, worin das zu untersuchende Nukleinsäuremolekül, und das Nukleinsäuremolekül kodierend für das Kil-Protein des Phagen Lambda, eingeführt wurde, wird in einem Mangelmedium für anorganisches Phosphat kultiviert, wobei das Mangelmedium das Substrat der ausgewählten Phosphatase-Aktivität vorzugsweise als einzige Phosphatquelle enthält und wobei das Substrat der ausgewählten Phosphatase-Aktivität, vorzugsweise in ausreichender Menge, um prinzipiell ein Wachstum der Wirtszellenkultur zu ermöglichen oder um den Phosphatbedarf der Wirtszellen zu decken, in dem Mangelmedium vorhanden ist;
d) das Wachstum der Wirtszellen in dem Medium wird nachgewiesen, wobei in Gegenwart des Nukleinsäuremoleküls, welches für die ausgewählte Phosphatase-Aktivität kodiert, Wachstum der Wirtszellen auftritt, und dieses Wachstum die Gegenwart eines Nukleinsäuremoleküls kodierend für die ausgewählte Phosphatase anzeigt.

Die Erfindung stellt somit ein Verfahren bereit, womit Gene, die eine bestimmte Phosphatase-Aktivität kodieren, aus einer Vielzahl verschiedener Organismen herausgefunden werden können. Das erfindungsgemäße Verfahren sieht dabei vor, in eine Wirtszelle sowohl das zu untersuchende Nukleinsäuremolekül als auch zusätzlich ein Nukleinsäuremolekül, das das Kil-Protein des Phagen Lambda kodiert, einzuführen, so dass die die beiden eingeführten Nukleinsäuremoleküle in der Wirtszelle exprimiert werden. Sollte das zu untersuchende Nukleinsäuremolekül ein Protein mit der ausgewählten Phosphatase-Aktivität kodieren, so wird dieses in dieser Wirtszelle exprimiert. Daneben, das heißt parallel dazu, bevorzugt gleichzeitig und/oder unter den gleichen Bedingungen, wird erfindungsgemäß das Kil-Protein exprimiert. Wird die Protein exprimierende Wirtszelle in dem erfindungsgemäßen Mangelmedium kultiviert, so zeigt das Wachstum der Wirtszelle das Vorhandensein eines Nukleinsäuremoleküls kodierend für die ausgewählte Phytaseaktivität in besonders deutlicher, das heißt in besonders sensitiver und selektiver Weise an. Besonders vorteilhaft ist daher, dass die Schritte des erfindungsgemäßen Verfahrens, besonders die Schritte c) und d), in automatisierter Weise durchgeführt werden können, beispielsweise in einem automatisierten Zellkultursystem, das besonders auch den automatisierten Nachweis des Wachstums der Wirtszellen ermöglicht.

In einer bevorzugten Ausführungsform des erfindungsgemäßen Verfahrens wird das zu untersuchende Nukleinsäuremolekül und bevorzugt auch das Nukleinsäuremolekül kodierend für das Kil-Protein des Phagen Lambda zunächst in einen Expressionsvektor kloniert (Schritt b1). Anschließend wird die Wirtszelle mit dem Expressionsvektor transformiert (Schritt b2). In einer besonders bevorzugten Variante wird zunächst ein das *kil*-Gen tragende Fragment aus der genomischen DNA des Phagen Lambda amplifiziert und in einen Vektor (Plasmid) eingeführt, insbesondere kloniert. In einem anderen Schritt wird das zu untersuchende Nukleinsäuremolekül bereitgestellt, indem es beispielsweise aus einem die Phosphatase-Aktivität exprimierenden Organismus, besonders einer Zelle, isoliert und mit geeigneten Primern amplifiziert wird. Danach wird das amplifizierte zu untersuchende Nukleinsäuremolekül in einem zweiten Vektor (Plasmid) eingeführt, insbesondere kloniert. Bevorzugt stehen die eingeführten Nukleinsäuremoleküle beziehungsweise Genabschnitte in den Vektoren unter der Kontrolle induzierbarer Promotoren. Anschließend wird der das *Kil*-Gen tragende Abschnitt, besonders als komplette Expressionskassette, des ersten Vektors, bevorzugt mittels Restriktionsenzymverdau, in den zweiten Vektor eingeführt. Es wird ein Expressionsvektor erhalten, der sowohl ein exprimierbares zu untersuchendes Nukleinsäuremolekül als auch bevorzugt ein exprimierbares Nukleinsäuremolekül kodierend für das Kil-Protein des Phagen Lambdas trägt.

Das erfindungsgemäße Verfahren lässt sich insbesondere dann besonders gut durchführen und löst die zugrunde liegende Aufgabe in besonderer Weise, wenn in der erfindungsgemäß bereitgestellten Wirtszelle nicht nur das zu untersuchende Nukleinsäuremolekül, sondern auch ein Nukleinsäuremolekül kodierend das Kil-Protein des Bakteriophagen Lambda besonders gleichzeitig, exprimiert wird.

Die vorliegende Erfindung betrifft auch die Wirtszellen und auch solche Zellen, die von der Wirtszelle abstammen, die mit den vorstehend genannten erfindungsgemäßen Nucleinsäuren und/oder Vektoren transformiert wurde.

Erfindungsgemäß bevorzugt liegt das zu untersuchende Nukleinsäuremolekül also in einem Expressionssystem vor und seine Expression steht unter der Kontrolle eines induzierbaren Promotors. Erfindungsgemäß besonders bevorzugt liegt auch das Nukleinsäuremolekül, welches für das Kil-Protein des Phagen Lambda kodiert, in einem Expressionssystem vor und seine Expression steht vorzugsweise unter der Kontrolle eines induzierbaren Promotors. Selbstverständlich können an Stelle der induzierbaren Promotoren auch konstitutive Promotoren eingesetzt werden, ohne die Lehre der Erfindung zu verlassen.

In einer bevorzugten Ausführungsform wird das Nukleinsäuremolekül, das für das Kil-Protein des Phagen Lambda kodiert, aus der genomischen DNA des Phagen Lambda mittels der Amplifikation durch die Polymerase-Kettenreaktion (PCR) mit einem Primerpaar mit den SEQ ID NO: 1 und SEQ ID NO: 2 erhalten. Dieses Nukleinsäuremolekül hat eine Länge von 287 Basenpaaren (bp). Selbstverständlich kann ein Kil-Protein kodierendes Nukleinsäuremolekül auch mit einem anderen Verfahren erhalten werden, beispielsweise mittels Synthese, Klonierung, Präparation etc.

Durch die erfindungsgemäß bevorzugte parallele Expression des *Kil-*Gens in der Wirtszelle wird ein "bacterial release"-Protein synthetisiert, das den Durchtritt von Proteinen aus dem intrazellulären Medium, insbesondere dem Periplasma, in den Extrazellulärraum ermöglicht. In gewisser Weise stellt eine solche Expressionskassette mit dem *Kil*-Gen ein induzierbares Sekretions- oder Lysesystem dar.

Ohne an die Theorie gebunden zu sein, erlaubt die Expression des *Kil*-Gens, besonders überraschend, die Sezemierung intrazellulär lokalisierter Proteine mit Phosphatase-Aktivität in das Extrazellulärmedium. In einer besonders bevorzugten Variante ist daher auch die Ko-Kultivierung von ersten Phosphatase und bevorzugt *Kil*-Gen exprimierenden Zellen und zweiten, insbesondere anderen, Phosphatase-defizienten Zellen beziehungsweise Zellen, die keine Phosphatase-Aktivität exprimieren, vorgesehen. Durch die im Falle einer positiven Probe erfindungsgemäß auftretende extrazelluläre Phosphatase-Aktivität werden auch die zweiten, insbesondere anderen, Phosphatase-defizienten Zellen mit anorganischem Phosphat versorgt, und können ebenfalls wachsen.

In besonders bevorzugter Ausführungsform ist die Phosphatase-defiziente Wirtszelle eine gram-negative prokaryotische Zelle, besonders bevorzugt eine Enterobakterien-Zelle. Besonders bevorzugt ist die erfindungsgemäße Wirtszelle *Escherichia coli*-Zelle (*E. coli*). Bevorzugt wird die Phosphatase-Defizienz dadurch erzeugt, dass die Transkription des PHO-Regulons der Zelle, besonders *E. coli*, unterbunden wird. Dies geschieht erfindungsgemäß bevorzugt durch Deletion des *phoB*-Gens. Besonders überraschend wird in der so erzeugten Phosphatase-defizienten Mutante eine globale down-Regulierung des PHO-Regulons erzielt. Dies führt vorteilhafterweise dazu, dass die so erzeugte Phosphatase-defiziente Mutante über keinerlei Phosphatase-Aktivität mehr verfügt und nur noch auf Medium mit ausreichender Menge an anorganischem ortho-Phosphat wachsen kann; dieses Phänomen wird vorliegend auch unter "Phosphatase-Defizienz" subsumiert.

In einer bevorzugten Ausführungsform wird das erfindungsgemäße Verfahren eingesetzt, um eine bestimmte Phosphatase-Aktivität zu selektieren, nämlich die Phosphatase-Aktivität eines gram-negativen Bakteriums. Bevorzugt ist die ausgewählte Phosphatase eine intrazelluläre, besonders cytoplasmatische oder periplasmatische, Phosphatase. Ohne an die Theorie gebunden zu sein, kodiert das Expressionsplasmid die Signalsequenz (z.B. Signalpeptid von OmpA, welche die Translokation der Phosphatase in das Periplasma über das sec-System erlaubt). In einer bevorzugten Variante ist die ausgewählte Phosphatase eine Phytase. Dabei wird erfindungsgemäß in Schritt c) des Verfahrens ein Mangelmedium gewählt, welches als Substrat Phytinsäure und/oder Phytat enthält. Das erfindungsgemäße Verfahren erlaubt in dieser bevorzugten Ausführungsform daher die besonders einfache und auch automatisierbare Selektion eines eine Phytase-Aktivität kodierenden Nukleinsäuremoleküls aus einer Gruppe zu untersuchender Nukleinsäuremoleküle, besonders aus einer Bibliothek beziehungsweise Bank von Nukleinsäuremolekülen.

Besonders überraschend gelingt mit dem erfindungsgemäßen Verfahren nicht nur der unmittelbare Nachweis einer ausgewählten Phosphatase-Aktivität, sondern es erlaubt auch eine quasi quantitative Bestimmung der ausgewählten Phytase-Aktivität. So gibt die beobachtete Intensität des Wachstums der die Phytase-Aktivität exprimierenden Wirtszellen einen direkten Aufschluss über die Aktivität der exprimierten Phosphatase. So spiegelt sich eine hohe Enzymaktivität in einem schnellen Wachstum beziehungsweise einer hohen Vermehrungsrate der Wirtszellen wieder.

In Schritt c) des erfindungsgemäßen Verfahrens werden die Wirtszellen in einem Mangelmedium kultiviert, welches das Phosphatase-Substrat als einzige Phosphatquelle enthält. Bevorzugt ist das Substrat in dem Mangelmedium in einer Konzentration von 0,1 bis 0,5 mmol/l, besonders bevorzugt von 0,15 bis 0,4 mmol/l, enthalten. Bevorzugt werden die Wirtszellen in dem Mangelmedium für einen Zeitraum von 2 bis 4 Tagen, besonders bevorzugt für 3 Tage, kultiviert. Nach dieser Kultivierungsdauer lassen sich deutliche Unterschiede im Wachstum der zu testenden Wirtszellen nachweisen. Die Kultivierungstemperatur beträgt vorzugsweise von 28 bis 32 °C, besonders bevorzugt 30 °C.

Schließlich ist ein weiterer Gegenstand der vorliegenden Erfindung die Verwendung des Nukleinsäuremoleküls, das für das Kil -Protein des Phagen Lambda kodiert, zum Zwecke des Auffindens von Nukleinsäuremolekülen, die für eine bestimmte Phosphatase-Aktivität kodieren in Selektionsverfahren, wobei insbesondere das vorstehend beschriebene erfindungsgemäße Verfahren durchgeführt wird.

Die Erfindung wird durch die beigefügten Figuren und Beispiele näher erläutert, wobei diese nicht beschränkend zu verstehen sind.

Die Figuren zeigen:
Figur 1, Beispiel einer Phosphatase-Aktivität: Die Phytase katalysierte Hydrolyse von Phytat zu Inositol und ortho-Phosphat in Gegenwart zweiwertiger Elemente;

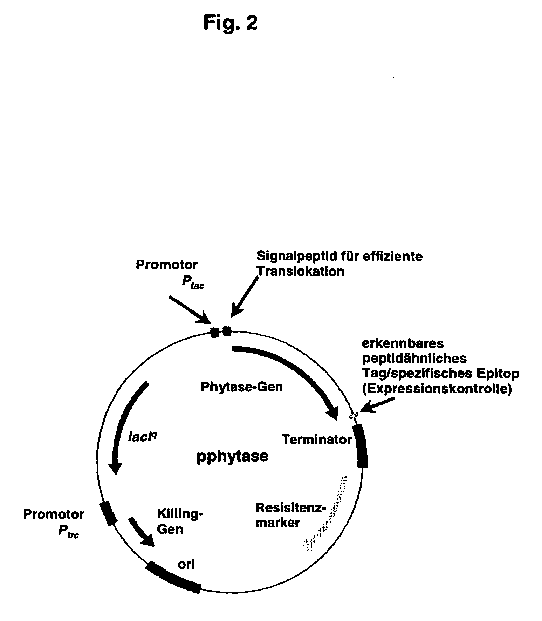

Figur 2, Schematische Darstellung eines Expressionsvektors enthaltend ein Nukleinsäuremolekül (Gen) kodierend eine Phosphatase-Aktivität (hier: Phytase-Aktivität) unter Kontrolle des Promotors P_{tac} und einen das *Kil*-Gen tragenden Abschnitt unter der Kontrolle des Promotors P_{trc}; sowie ein N-terminales Signalpeptid und ein C-terminales Flag-Tag.

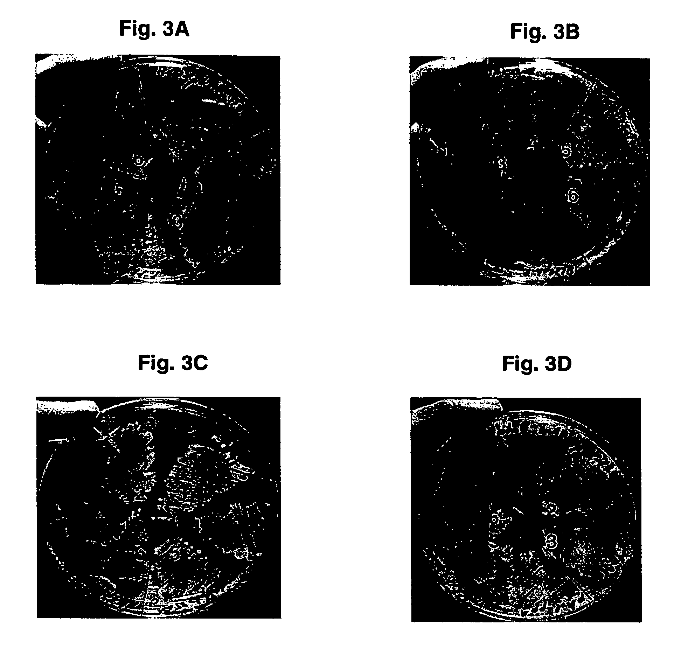

Figuren 3A, 3B, 3C und 3D, Analyse des Wachstums von *E, coli-*Kulturen des Stamms BL21 *phoB*::Tn10, ausgestrichen in Petrischalen; Figur 3A, Zellen nach Transformation des Stamms mit dem Plasmid pMCS sowie davon abgeleitete Zellen auf Minimalmedium enthaltend eine angemessene Menge an anorganischem Phosphat; Figur 3B, gleiche Organismen wie in Figur 3A, Wachstum auf Minimalmedium, enthaltend Phytat als einzige Phosphorquelle; Figur 3C, Zellen nach Transformation mit dem Plasmid pkil sowie davon abgeleitete Zellen, Wachstum auf Minimalmedium enthaltend eine angemessene Menge an anorganischem Phosphat; Figur 3D, gleiche Organismen wie in Figur 3C, Wachstum auf Minimalmedium, enthaltend Phytat als einzige Phosphorquelle; Legende: (1) Kontroll-*phoB-*Stamm; (2) Kontrolle-phoB-Stamm enthaltend das Eltemplasmid; (3) *phoB*-Stamm, welcher die plasmidkodierte Phytase II von *E. coli* exprimiert; (4) *phoB*-Stamm, welcher die plasmidkodierte Phytase I von *E. coli* exprimiert; (5) *phoB*-Stamm, welcher das plasmidkodierte PhyA von *Bacillus amyloliquefaciens FZB45* codiert und worin zwischen *phyA* und dem C-terminalen Flag-Tag ein Stop-Kodon eingefügt ist; (6) *phoB*-Stamm, welcher das plasmidkodierte PhyA von *Bacillus amyloliquefaciens FZB45* exprimiert und worin *phyA* C-terminal mit Flag-Tag fusioniert ist;
Figur 4, Klonierung des *Kil*-Gens in das Plasmid pCrcHis2/lacZ unter der Kontrolle des induzierbaren P_{trc}-Promotors;
Figur 5, Klonierung des *appA*-Gens beziehungsweise des *agp*-Gens unter der Kontrolle des induzierbaren P_{tac}-Promotors;
Figuren 6A, 6B und 6C, Einführen von P_{trc}kil in Plasmid pMCS um pkil zu erhalten (Figur 6A), in Plasmid pappA, um pkilappA zu erhalten (Figur 6B) und in Plasmid pagp, um pkilagp zu erhalten (Figur 6C);
Figur 7, Klonierung des *phyA*-Gens in das Plasmid pMCS unter der Kontrolle des induzierbaren Promotors P_{tac};
Figur 8, Klonierung des *phyA*-Gens in das Plasmid pkil unter der Kontrolle des induzierbaren Promotors P_{tac};
Figuren 9A und 9B, Westerblot-Analyse: Auftrennung von Zellextraktproben (Figur 9A), Auftrennung von Proben aus dem überstehenden Medium (Figur 9B); Legende: (1) MG1655 (wt); (2) MG1655 induziert mit IPTG; (3) Marker; (4) MG1655 pMCS; (5) MG1655 pMCS induziert mit IPTG; (6) MG1655 pkil; (7) MG1655 pkil induziert mit IPTG; (8) MG1655 pappA; (9) MG1655 pappA induziert mit IPTG; (10) MG1655 pkilappA; (11) MG1655 pkilappA induziert mit IPTG; (12) MG1655 pagp; (13) MG1655 pagp induziert mit IPTG; (14) MG1655 pkilagp; (15) MG1655 pkilagp induziert mit IPTG;

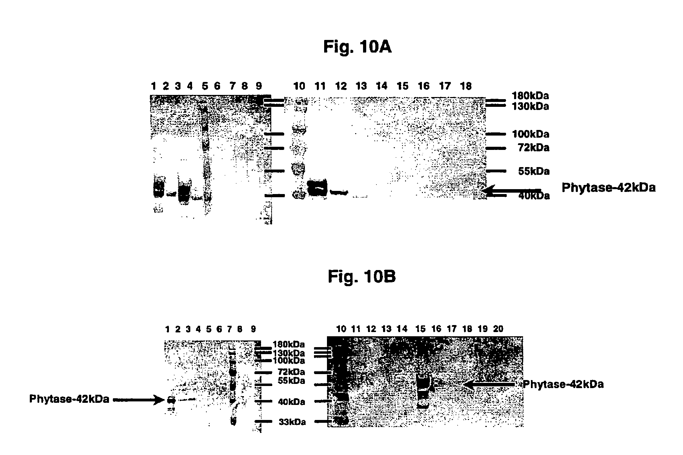

Figuren 10A und 10B, Westemblot-Analyse der Zellextraktproben für pMCS und die abgeleiteten Zellen unter Verwendung eines primären polyklonalen *anti*-phyA Antikörpers und eines sekundären polyklonalen *anti*-rabbit IgG AP-konjugierten Antikörpers (Spur 1 bis 9) oder eines monoklonalen *anti*-Flag-Tag M2 Antikörpers (Spur 10 bis 18 in Figur 10A, Spur 10 bis 20 in Figur 10B); Legende zu Figur 10A: (1) und (11) MG1655 *phoB*::Tn10 pphy45Flag induziert mit IPTG; (2) und (12) MG1655 *phoB*::Tn10 pphy45Flag; (3) und (13) MG1655 *phoB*::Tn10 pphy45STOP induziert mit IPTG; (4) und (14) MG1655 *phoB*::Tn10 pphy45STOP; (5) und (10) Marker; (6) und (15) MG1655 *phoB*::Tn10 pMCS induziert mit IPTG; (7) und (16) MG1655 *phoB*::Tn10 pMCS; (8) und (17) MG1655 *phoB*::Tn10 induziert mit IPTG; (9) und (18), MG1655 *phoB*::Tn10; Legende für Figur 10B: (1) BL21 *phoB* pkilphy45Flag induziert mit IPTG; (2) BL21 *phoB* pkilphy45Flag induziert mit Lactose; (3) BL21 *phoB* pkilphy45Flag; (4) BL21 *phoB* pkilphy45STOP induziert mit IPTG; (5) BL21 *phoB* pkilphy45STOP induziert mit Lactose; (6) BL21 *phoB:*Tn10 pkilphy45STOP; (7) Marker; (8) BL21 *phoB*:Tn10 pkil induziert mit IPTG; (9) BL21 *phoB*:Tn10 pkil induziert mit Lactose; (10) Marker; (11) BL21 *phoB*::Tn10 pkilphy45STOP induziert mit IPTG; (12) BL21 *phoB*::Tn10 pkilphy45STOP; (13) BL21 *phoB*::Tn10 pkil induziert mit IPTG; (14) BL21 *phoB*::Tn10 pkil; (15) MG1655 *phoB*::Tn10 pkilphy45Flag induziert mit IPTG; (16) MG1655 *phoB*::Tn10 pkilphy45Flag; (17) MG1655 *phoB*::Tn10 pkil induziert mit IPTG; (18) MG1655 *phoB*::Tn10 pkil; (19) MG1655 *phoB*::Tn10 induziert mit IPTG; (20) MG1655 *phoB*::Tn10.

### Beispiel 1: Einführen des Kil-Gens des Bacteriophagen Lambda in das Plasmid pTrcHis2/lacZ

Ein Plasmid wurde konstruiert, um eine induzierbare Expression des Kil-Proteins zu erhalten. Dadurch kann eine hohe und kontrollierbare Zelllyse einer gram-negativen Wirtszelle wie *E. coli* erreicht werden.

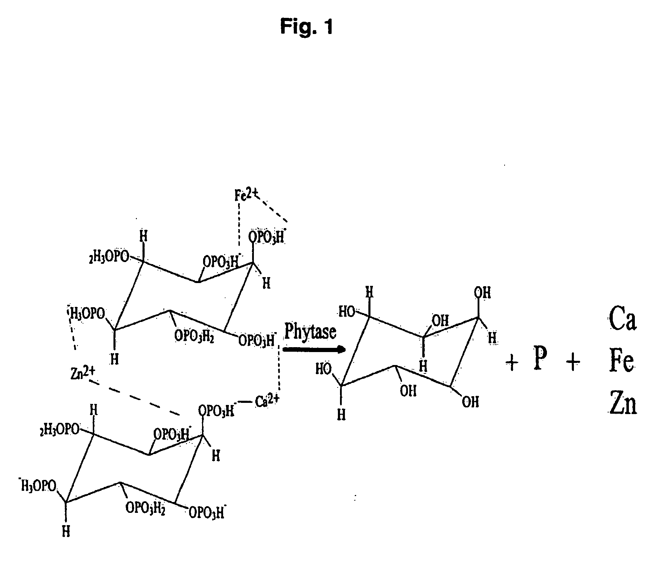

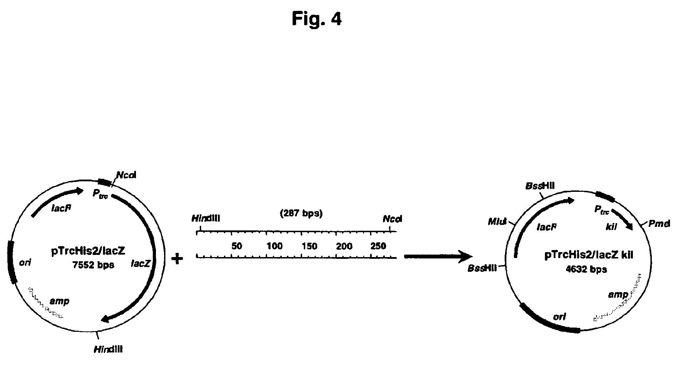

Aus präparierter genomischer DNA des Bacteriophagen Lambda wurde ein 287 bp DNA-Fragment amplifiziert, das das Kil-Protein kodiert. Dazu wurden die Primer SEQ ID NO: 1 und SEQ ID NO: 2 (Tabelle 1) verwendet. Die PCR-Fragmente wurden verdaut und in das Plasmid pTrcHis2/lacZ (Figur 4) als *Nco*I*-Hind*III Fragmente kloniert, um das Plasmid pTrcHis2/lacZ kil (Figur 1) zu erhalten. Die PCR-Bedingungen waren (Temperatur in °C/Zeit in Minuten):(98/10), (96/0,75;55/0,50;72/1,5)_{35,} (72/10); Enzym: Herculase.

Zwölf der erhaltenen Klone wurden mittels Restriktionsverdau mit *Bsa*Al analysiert. Die erwarteten Fragmentlängen betrugen für pTrcHis2/lacZ: 7532 bps und für pTrcHis2/lacZ kil: 1894 bps und 2738 bps. Drei von zwölf getesteten Klonen wiesen das richtige Au f-trennungsprofil auf.

**Tabelle 1:**

| **Primer [SEQ ID NO.]** | | **Sequenz** |
|---|---|---|
| 1 | kilNcoIhin | CATGCCATGGCATGCCATTGCAGGGTGGCCTGT |
| 2 | kilHindIIher | CCCAAGCTTGTGAATGCTTTTGCTTGATCTCAG |
| 3 | appaXhoIhin | CCGCTCGAGCGAAAGCGATCTTAATCCCATTT |
| 4 | appaBgIIIher | GAAGATCTTCCCAAACTGCACGCCGGTATGCGT |
| 5 | agpXhoIhin | CCGCTCGAGCGAACAAAACGCTAATCGCCGCAG |
| 6 | agpBgIIIher | GAAGATCTTCCTTTCACCGCTTCATTCAACACG |
| 7 | 45hinXhoI | CCGCTCGAGCGAAGCATAAGCTGTCTGATCCTTAT |
| 8 | 45herBgIIISTOP | GGAAGATCTTTATTTTCCGCTTCTGTCGGTCAG |
| 9 | 45herBgIIIFlag | GGAAGATCTTTTTCCGCTTCTGTCGGTCAGTTT |

### Beispiel 2: Klonieren des appA-Gens und des agp-Gens von E. coli

Es wurden Plasmide konstruiert, um eine hohe und kontrollierte Expression von AppA (Phytase I) und Agp (Glucose-1-Phosphatase beziehungsweise Phytase II) zu erhalten.

### a) Klonieren von appA

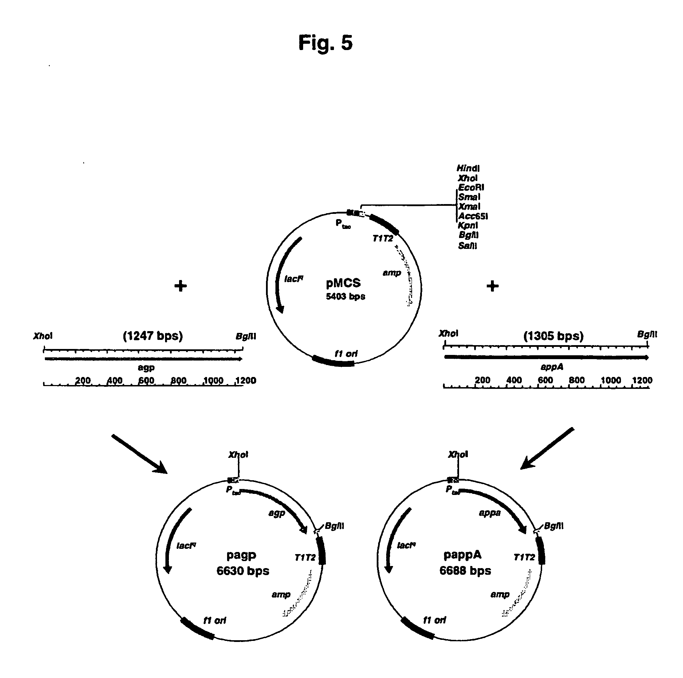

Aus präparierter genomischer DNA aus dem *E. coli* Stamm MG 1655 wurde ein 1316 bp DNA Fragment amplifiziert, das *appA* Gen, das für eine saure Phosphatase (Phytase I) kodiert. Dazu wurden die Primer SEQ ID NO: 3 und SEQ ID NO: 4 (Tabelle 1) verwendet. Die PCR-Fragmente wurden verdaut und als *Xho*I-*Bg*/II Fragmente kloniert, um das Plasmid pappA (Figur 5) zu erhalten.

Zwei der erhaltenen Klone wurden mittels Restriktionsverdau mit *Sa*/I analysiert. Die erwarteten Fragmentlängen betrugen für pMCS: 5403 bps und für pagp: 1081 bps und 5546 bps. Einer der zwei getesteten Klone wiesen das korrekte Auftrennungsprofil auf.

### b) Klonieren von agp

Aus präparierter genomischer DNA aus dem *E. coli* Stamm MG 1655 wurde ein 1258 bp DNA Fragment amplifiziert, das agp-Gen, das für eine saure Phosphatase (Phytase II) kodiert. Dazu wurden die Primer SEQ ID NO: 5 und SEQ ID NO: 6 (Tabelle 1) verwendet. Die PCR-Fragmente wurden verdaut und als *Xho*I-*Bg*/II Fragmente kloniert, um das Plasmid pagp (Figur 5) zu erhalten.

Elf der erhaltenen Klone wurden mittels Restriktionsverdau mit *Sa*/I analysiert. Die erwarteten Fragmentlängen betrugen für pMCS: 5403 bps und für pagp: 975 bps und 5713 bps. Drei von elf der getesteten Klone wiesen das korrekte Auftrennungsprofil auf.

### Beispiel 3: Einführen von P_{trc}kil in die Plasmide pMCS, pappA, pagp

### a) Einführen von P_{trc}kil in Plasmid pMCS

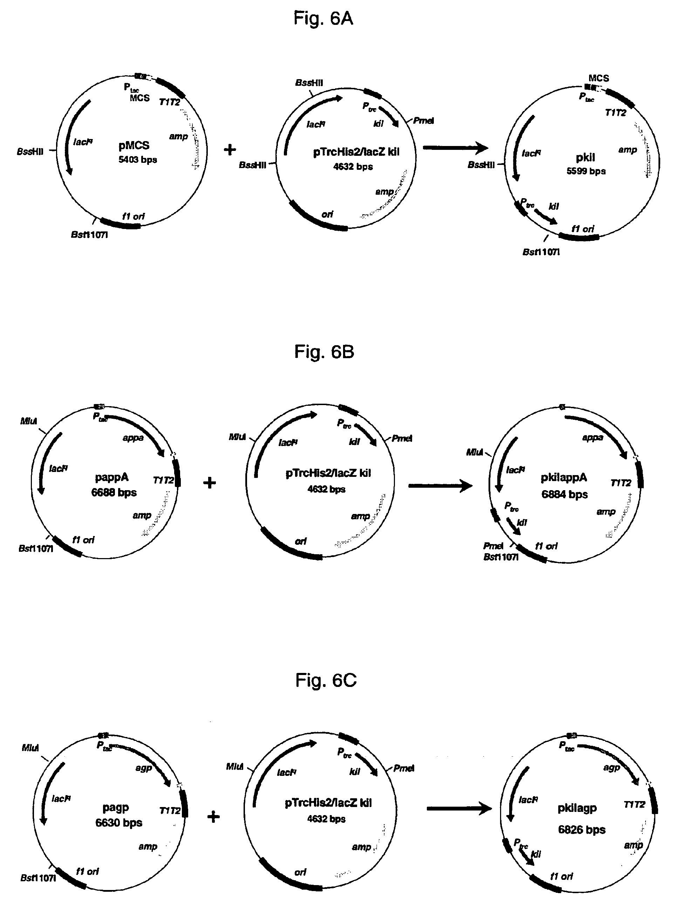

Das Plasmid pTrcHis2/lacz kil (siehe Beispiel 1) wurde mit den Restriktionsenzymen *Pme*I und *Bss*HII verdaut, wobei ein DNA-Fragment mit 1126 bps isoliert wurde. Plasmid pMCS wurde mit den Restriktionsenzymen *Bss*HII und *Bst*11071 verdaut, wobei ein DNA-Fragment mit 4477 bps isoliert wurde. Diese PCR-Fragmente wurden kloniert, um das Plasmid pkil (Figur 6A) zu erhalten.

Zwölf der erhaltenen Klone wurden mittels Restriktionsverdau mit *Nco*I analysiert. Die erwarteten Fragmentlängen betrugen für pTrcHis2/lacz kil: 4632 bps, für pMCS: 5408 bps und für pkil: 1606 bps und 3993 bps). Alle zwölf getesteten Klone wiesen das korrekte Auftrennungsprofil auf.

### b) Einführen von P_{trc}kil in Plasmid pappA

Das Plasmid pTrcHis2/lacz kil (siehe Beispiel 1) wurde mit den Restriktionsenzymen *Pme*I und *Mlu*I verdaut, wobei ein DNA-Fragment mit 1537 bps isoliert wurde. Plasmid pappA wurde mit den Restriktionsenzymen *Mlu*I und *Bst*11071 verdaut, wobei ein DNA-Fragment mit 5519 bps isoliert wurde. Diese PCR-Fragmente wurden kloniert, um das Plasmid pkilappA (Figur 6B) zu erhalten.

Zwölf der erhaltenen Klone wurden mittels Restriktionsverdau mit *NcoI* analysiert. Die erwarteten Fragmentlängen betrugen für pTrcHis2/lacz kil: 4632 bps, für pappA: 6688 bps und für pkilagp: 1606 bps und 5278 bps). Sieben der zwölf getesteten Klone wiesen das korrekte Auftrennungsprofil auf.

### c) Einführen von P_{trc}kil in Plasmid pagp

Das Plasmid pTrcHis2/lacz kil (siehe Beispiel 1) wurde mit den Restriktionsenzymen *Pme*I und *Mlu*I verdaut, wobei ein DNA-Fragment mit 1537 bps isoliert wurde. Plasmid pagp wurde mit den Restriktionsenzymen *Mlu*I und *Bst*11071 verdaut, wobei ein DNA-Fragment mit 5351 bps isoliert wurde. Die PCR-Fragmente wurden kloniert, um das Plasmid pkilagp (Figur 6C) zu erhalten.

Zwölf der erhaltenen Klone wurden mittels Restriktionsverdau mit *Nco*I analysiert. Die erwarteten Fragmentlängen betrugen für pTrcHis2/lacz kil: 4632 bps, für pagp: 6630 bps und für pkilagp: 1606 bps und 5220 bps). Acht der zwölf getesteten Klone wiesen das korrekte Auftrennungsprofil auf.

### Beispiel 4: Einführen des phyA-Gens aus Bacillus amyloliauefaciens FZB45 in das Plasmid pMCS

Das Plasmid pMCS ermöglicht über den Promotor P_{tac} eine starke und steuerbare Expression. Das darin vorkommende C-terminale "Flag-Tag" erleichtert Aufreinigung und Nachweis des exprimierten Target-Proteins mittels eines *anti*-Flag Antikörpers. Der C-Terminus ist jedoch im vorliegend gewählten Beispiel auf das Zentrum des Phytaseproteins PhyA gerichtet, wobei das Protein eine stark komplex gefaltete β-Propellerstruktur aufweist. Um den möglichen Einfluss der Fusion des *phyA*-Gens mit dem Plasmid kodierten "Flag-Tag" auf die richtige Faltung des Proteins oder auf die Hemmung der Enzymaktivität auszuschließen, wurden zwei Klonierungsstrategien entwickelt: Einmal wurde *phyA*-Gen im Expressionsrahmen des "Flag-Tags" kloniert; in einem zweiten Ansatz wurde ein Stop-Codon zwischen das *phyA*-Gen und das Flag-Tag eingefügt.

Dementsprechend wurden Plasmide konstruiert, die eine hohe und kontrollierbare Expression der Phytase aus *Bacillus amyloliquefaciens FZB45* (PhyA) in einer Wirtszelle wie *E. coli* ermöglichen.

### a) Ansatz PhyA im Flag-Tag Expressionsrahmen

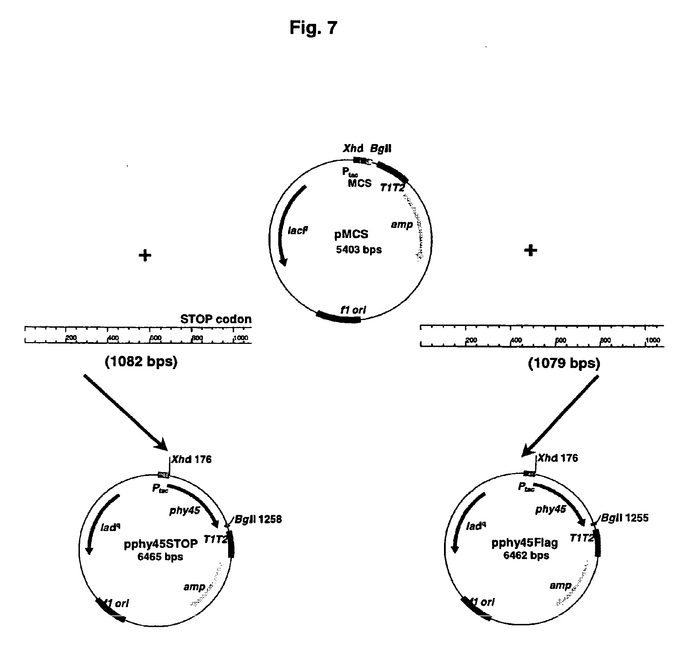

Aus der präparierten genomischen DNA aus *Bacillus amyloliquefaciens FZB45* wurde ein 1082 bp DNA-Fragment amplifiziert, welches das PhyA-kodiert. Dazu wurden die Primer SEQ ID NO: 7 und SEQ ID NO: 8 (Tabelle 1) eingesetzt. Die PCR-Fragmente wurden verdaut und in Form eines *Xho*I-*Bgl*II-Fragments in das Plasmid pMCS kloniert, um das Plasmid pphy45STOP (Figur 7) zu erhalten. Die PCR-Bedingungen waren (Temperatur in °C/Zeit in Minuten): (95/5) (95/0,5; 58/0.5; 72/1,5)₃₅, (72/10); Enzym: Herculase.

30 der erhaltenen Klone wurden über die PCR unter Verwendung der Primer SEQ ID NO: 7 und SEQ ID NO: 8 analysiert. Sechs positive Klone wurden mittels Restriktionsverdau mit *Eco*47III weiter untersucht. Die erwarteten Fragmentlängen betrugen für pMCS: 5403 bps und für pphy45STOP: 5388 bps und 1077 bps. Vier der sechs getesteten Klone wiesen das korrekte Auftrennungsprofil auf.

### b) Ansatz mit Stop-Codon

Aus der präparierten genomischen DNA aus *Bacillus amyloliquefaciens FZB45* wurde ein 1079 bp DNA-Fragment amplifiziert, welches PhyA kodiert. Dazu wurden die Primer SEQ ID NO: 7 und SEQ ID NO: 9 (Tabelle 1) eingesetzt. Die PCR-Fragmente wurden verdaut und in Form eines *Xho*I-*Bgl*II-Fragments in das Plasmid pMCS kloniert, um das Plasmid pphy45Flag (Figur 7) zu erhalten. Die PCR-Bedingungen waren (Temperatur in °C/Zeit in Minuten): (95/5) (95/0,5; 58/0,5; 72/1,5)_{35,} (72/10); Enzym: Herculase.

38 der erhaltenen Klone wurden über die PCR unter Verwendung der Primer SEQ ID NO: 7 und SEQ ID NO: 9 analysiert. Sechs positive Klone wurden mittels Restriktionsverdau mit *Eco*47III weiter untersucht. Die erwarteten Fragmentlängen betrugen für pMCS: 5403 bps und für pphy45Flag: 5385 bps und 1077 bps. Alle sechs getesteten Klone wiesen das korrekte Auftrennungsprofil auf.

### Beispiel 5: Einführen des phyA-Gens aus Bacillus amyloliauefaciens FZB45 in das Plasmid pkil

Diese Plasmide wurden konstruiert, um eine hohe und kontrollierte Expression der Phytase aus *Bacillus amylotiquefaciens FZB45* (PhyA) in Wirtszellen wie *E. coli* zu erzielen. Diese Plasmide ermöglichen die kontrollierte Freisetzung des exprimierten Enzyms in den extrazellulären Raum. Um den Einfluss des Flag-Tags auszuschalten (siehe Beispiel 4), wurden zwei alternative Klonierungsstrategien entwickelt:

### a) Ansatz PhyA im Flag-Tag Expressionsrahmen

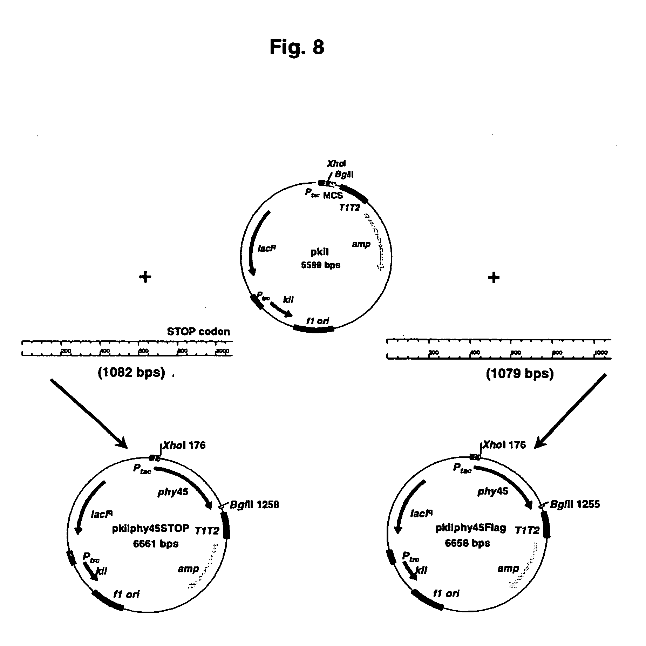

Aus der präparierten genomischen DNA aus *Bacillus amyloliquefaciens FZB45* wurde ein 1082 bp DNA-Fragment amplifiziert, welches das PhyA-kodiert. Dazu wurden die Primer SEQ ID NO: 7 und SEQ ID NO: 8 (Tabelle 1) eingesetzt. Die PCR-Fragmente wurden verdaut und in Form eines *Xho*I-*Bgl*II-Fragment in das Plasmid pMCS kloniert, um das Plasmid pkilphy45STOP (Figur 8) zu erhalten. Die PCR-Bedingungen waren (Temperatur in °C/Zeit in Minuten): (95/5) (95/0,5; 58/0.5; 72/1,5)_{35,} (72/10); Enzym: Herculase.

Acht der erhaltenen Klone wurden über die PCR unter Verwendung der Primer SEQ ID NO: 7 und SEQ ID NO: 8 analysiert. Fünf positive Klone wurden mittels Restriktionsverdau mit *Eco*47III weiter untersucht. Die erwarteten Fragmentlängen betrugen für pkil: 5599 bps und für pkilphy45STOP: 5584 bps und 1077 bps. Alle fünf getesteten Klone wiesen das korrekte Auftrennungsprofil auf.

### b) Ansatz PhyA mit Stop-Codon

Aus der präparierten genomischen DNA aus *Bacillus amyloliquefaciens FZB45* wurde ein 1079 bp DNA-Fragment amplifiziert, welches das PhyA-kodiert. Dazu wurden die Primer SEQ ID NO: 7 und SEQ ID NO: 9 (Tabelle 1) eingesetzt. Die PCR-Fragmente wurden verdaut und in Form eines *Xho*I-*Bgl*II-Fragment in das Plasmid pMCS kloniert, um das Plasmid pkilphy45Flag (Figur 8) zu erhalten. Die PCR-Bedingungen waren (Temperatur in °C/Zeit in Minuten): (95/5) (95/0,5; 58/0,5; 72/1,5)_{35,} (72/10); Enzym: Herculase.

Fünf der erhaltenen Klone wurden über die PCR unter Verwendung der Primer SEQ ID NO: 7 und SEQ ID NO: 9 analysiert. Ein positiver Klon wurden mittels Restriktionsverdau mit *Eco*47III weiter untersucht. Die erwarteten Fragmentlängen betrugen für pkil: 5599 bps und für pkIIphy45Flag: 5581 bps und 1077 bps. Der getestete Kion wies das korrekte Auftrennungsprofil auf.

### Beispiel 6: Translation von agp und appA in Wirtszellen

### a) Kultivierung

Jeweils 10 µl von Übemachtkulturen der Bakterienstämme MG1655, MG1655 pMCS, MG1655 pkil, MG1655 pappA, MG1655 pkilappA, MG1655 pagp und MG1655 pagpkil (siehe Tabelle 2) wurden in 10 ml LB-Medium mit 100 µg/ml Ampicillin bei 37°C inokuliert. Während der mittleren log-Phase (OD₆₀₀: 0,4 - 0,7) wurde IPTG (Isopropylbeta-D-thiogalactopyranosid) in einer Endkonzentration von 1 mmol/l hinzugegeben. Dadurch wurde die Expression von AppA und Agp von dem Promotor P_{tac} aus und die Expression von Kil von dem Promotor P_{trc} aus induziert. Die Zellen wurden anschließend für 2,5 Stunden bei 37°C geschüttelt. Danach wurden die Zellkulturen zentrifugiert und die Zellpellets sowie die Überstände (Medium) bei - 20°C aufbewahrt.

### b) SDS-PAGE

Zur Zelllyse wurden die Probenpellets in 200 µl eines 1xSDS-PAGE Probenpuffers resuspendiert. Danach wurden (zur Extraktion) 20 µl CHCl₃ zugegeben. Zur Extraktion wurden die Proben vortexgerührt, zentrifugiert und die Überstände der Wasserphase mit Probenpuffer gemischt. Die Überstände (Medium) der Zellkulturen wurden ebenfalls mit Probenpuffer gemischt. Die Extraktproben und die Proben des Zellkulturüberstands wurde ein Lauf in SDS-PAGE (12%) aufgetrennt.

### c) Westem-Blot-Analyse

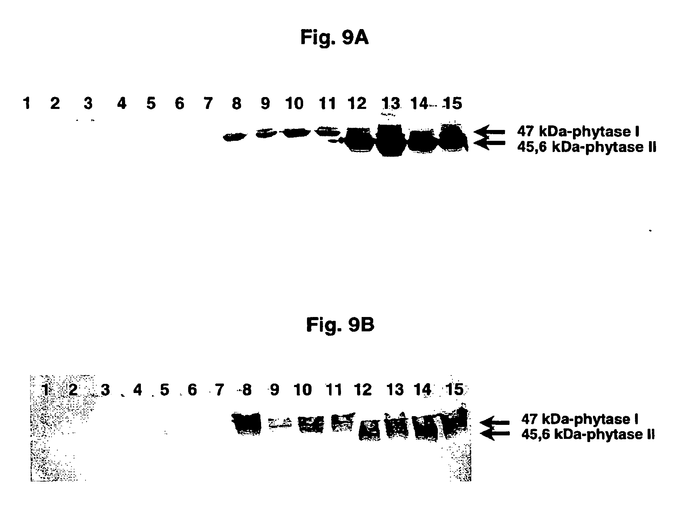

Die Proteine wurden auf Nitrozellulosemembran, unter Verwendung eines Transferpuffers mit 25 mmol/l Tris bei pH 8,3, 192 mmol Glycin sowie 20 Vol.-% Methanol übertragen (1 Stunde bis 1,5 Stunde bei 100 V und 4°C). Die Membran wurde entnommen und für 20 Minuten in phosphatgepufferter Saline (PBS) mit 5 % Milchpulver geblockt, mit Wasser gespült und anschließend, mit einem monoklonalen *anti-Flag* AP-konjugierten Antikörper (Verdünnung 1:2000; Sigma) in PBS versetzt mit 3 % Milchpulver, über Nacht bei Raumtemperatur inkubiert. Die Membran wurde dann dreimal für 15 Minuten mit PBS, welches 0,1 % Tween 20 enthielt, gewaschen. Danach wurde sie noch einmal 10 Minuten mit PBS gewaschen und mit dem "Alkaline Phosphatase Conjugated Substrate Kit" von BIO-RAD behandelt, siehe Figur 9A und Figur 9B.

### d) Ergebnis

In beiden Fällen wurde eine starke Expression des Target Proteins beobachtet. Weiter wurde eine effektive Sekretion beider Proteine in den extrazellulären Raum erreicht (Figuren 9A und 9B).

### Beispiel 7: Translation von phyA in Wirtszellen

### a) Kultivierung

Jeweils 10 µl von Übernachtkulturen der Bakterienstämme BL21 *phoB*::Tn10, BL21 *phoB*::Tn10 pMCS, BL21 *phoB*::Tn10 pphy45STOP, BL21 *phoB*::Tn10 pphy45Flag, BL21 *phoB*::Tn10 pkil, BL21 *phoB*::Tn10 pkilphy45STOP, und BL21 *phoB*::Tn10 pkilphy45Flag (siehe Tabelle 2) wurden in 10 ml LB-Medium mit 12,5 µg/ml Tetracyclin und 100 µg/ml Ampicillin bei 37°C inokuliert. Während der mittleren log-Phase (OD₆₀₀:0,7) wurde IPTG in einer Endkonzentration von 1 mmol/l hinzugegeben. Dadurch wurde die Expression von PhyA von dem Promotor P_{tac} aus und die Expression von Kil von dem Promotor P_{trc} aus induziert. Die Zellen wurden anschließend für zwei Stunden bei 37°C geschüttelt. Danach wurden die Zellkulturen zentrifugiert und Zellpellets und Überstände (Medium) bei -20°C aufbewahrt.

### b) SDS-PAGE

Zur Zelllyse wurden die Probenpellets in 200 µl eines 1xSDS-PAGE Probenpuffers resuspendiert. Danach wurden (zur Extraktion) 20 µl CHCl₃ zugegeben. Zur Extraktion wurden die Proben vortexgerührt, zentrifugiert und die Überstände der Wasserphase mit Probenpuffer gemischt. Die Überstände (Medium) der Zellkulturen wurden ebenfalls mit Probenpuffer gemischt. Die Extraktproben und die Proben des Zellkulturüberstands wurden in SDS-PAGE (12%) aufgetrennt.

### c) Westem-Blot-Analyse

Die Proteine wurde auf Nitrozellulosemembran unter Verwendung eines Transferpuffers mit 25 mmol/l Tris bei pH 8,3, 192 mmol/l Glycin sowie 20 Vol.% Methanol übertragen (1 Stunde bei 100 V und 4°C). Die Membran wurde entnommen und für 1 Stunde in Trisgepufferter Saline (TBS) mit 5 % Milchpulver geblockt, mit Wasser gespült und anschließend mit einem primären polyklonalen anti-PhyA-Antikörper (Verdünnung 1:3000) oder der einem monoklonalen AP-konjugierten *anti*-Flag-Antikörper (Verdünnung 1:2000; Sigma) in TBS versetzt mit 1 % bovinem Serumalbumin (BSA) über Nacht bei Raumtemperatur inkubiert. Die Membran wurde dreimal für 5 Minuten in TBS gewaschen. War ein sekundärer Antikörper erforderlich, so wurde die Membran mit *anti*-Rabbit IgG AP-konjugierten Antikörper (Verdünnung 1:1000; cell signaling) in TBS versetzt mit 1 % BSA, inkubiert. Danach wurde die Membran dreimal für 15 Minuten in TBS mit 0,1 % Tween 20 gewaschen, anschließend mit TBS gespült und mit dem "Alcaline Phosphatase Conjugated Substrate Kit" von BIO-RAD behandelt, siehe Figur 10A und Figur 10B.

### d) Ergebnisse

Die untersuchten Plasmide exprimieren das Protein PhyA aus *Bacillus amyloliquefaciens FZB45.* Sowohl der anti-PhyA Antikörper als auch der *anti*-Flag Antikörper sind in der Lage, das Fusionsprotein PhyA-Flag zu erkennen (siehe Figur 10A, Spur 1, 2, 11 und 12). Die Expression des PhyA-Proteins konnte mit dem *anti*-PhyA-Antikörpers nachgewiesen werden (siehe Figur 10A, Spur 3 und 4). Darüber hinaus wurde in Gegenwart von IPTG eine hohe Expression der Proteine nachgewiesen.

Die von pkil abgeleiteten untersuchten Plasmide exprimieren das PhyA-Protein aus *Bacillus amylotiquefaciens FZB45.* Sowohl der *anti*-PhyA Antikörper als auch der *anti*-Flag Antikörper sind in der Lage, das Fusionsprotein PhyA-Flag (siehe Figur 10B, Spur 1, 2, 3, 15 und 16) zu erkennen. Die Erkennung des nicht mit einem Tag-Peptid versehenen PhyA-Proteins mittels des *anti*-PhyA Antikörpers ist weniger deutlich (siehe Figur 10B, Spur 4,5 und 6).

### Beispiel 8: Konstruktion der phoB-Deletionsmutante

### a) Präparation des P1ᵥᵢᵣ-Lysats

Um ein P1ᵥᵢᵣ-Lysat aus dem Bakterienstamm HA15 herzustellen, wurden 10 µl einer Übemachtkultur des Stamms in 10 ml LB-Medium, welches 5 mmol/l CaCl₂ enthielt, bei 37 °C inokuliert. Während der exponentiellen Wachstumsphase wurden die Bakterienzeilen mit dem vorbereiteten P1ᵥᵢᵣ-Lysat aus dem Wildtyp MG1655 infiziert.

Die infizierte Kultur wurde weiter für 4 Stunden bei 37 °C inkubiert bis Zelllyse beobachtet wurde. Zur Extraktion wurde CHCl₃ hinzugegeben, das Lysat wurde zentrifugiert, der Überstand in ein neues Probenröhrchen mit CHCl₃ übertragen und bei 4 °C gelagert.

### b) P1-Transduktion

50 µl einer Übemachtkultur der Empfängerstämme BL21, TG1, IS568, ES1568, JM110, MG1655 *mutL*::Tn5 wurden in LB-Medium bei 37 °C inokuliert bis die exponentielle Wachstumsphase erreicht wurde. Jeweils 900 µl dieser Zellen wurden mit 2,5 µl CaCl₂ (1 mol/l) und 100 µl P1ᵥᵢᵣ-Lysat des Bakterienstammes HA15 (*phoB*::Tn10) versetzt. Die Phagenabsorption, die 38 Minuten bei 37 °C erfolgte, wurde durch die Zugabe von 5 mmol/ml Natriumcitrat in LB gestoppt.

Die Zellen wurden 1 Stunde bei 37 °C ohne Schütteln zur phänotypischen Expression inkubiert. Die Bakterienkultur wurde abzentrifugiert und auf Selektionsplatten, versetzt mit 12,5 µg/ml Tetracyclin ausplattiert. Die erhaltenen Einzelkolonien wurden aufgereinigt und über Nacht bei 37 °C inkubiert.

### Beispiel 9: Transformation kompetenter Zellen

Kompetente Zellen der Stämme MG1655, MG1655 *phoB*::Tn10, MG1655 *mutL*::Tn5, MG1655 *mutL*::Tn10 *phoB*::Tn10, BL21, BL21 *phoB*::Tn10, TG1, TG1 *phoB*::Tn10, ES568, ES568 *phoB*::Tn10, ES1578, ES1578 *phoB*::Tn10, JM110, JM110 *phoB*::Tn10 and DH5α (*phoA⁻*) wurden gemäß des Rubidiumchlorid-Protokolls (Fa. Promega) hergestellt. Schließlich wurden pro Probenröhrchen 200 µl aliquotiert und bei -80 °C gelagert.

Jeder Stamm wurde mit jeweils einem der folgenden Plasmide transformiert: pMCS, pkil, pappA, pkilappA, pagp and pkilagp. Außerdem wurden die Stämme MG1655 *phoB*::Tn10, TG1 *phoB*::Tn10 und BL21 *phoB*::Tn10 jeweils mit den Plasmiden pphy45STOP, pkylphy45Flag sowie pkilphySTOP transformiert.

Die jeweils erhaltenen Einzelkolonien wurden aufgereinigt und auf LB-Agarplatten oder Selektionsplatten, welcher 100 µg/ml Ampicillin enthielt, über Nacht bei 37 °C inkubiert. Die erzeugten Bakterienstämme sowie die davon abgeleiteten plasmidtragenden Derivate wurden mit Glyzerin versetzt und bei - 80 °C gelagert. Die Bakterienstämme sind in Tabelle 2 aufgeführt.

Der Stamm *Escherichia coli* BL21 *phoB*::*Tn*10 pappA wurde bei der Deutschen Sammlung von Mikroorganismen und Zellkulturen GmbH, Mascheroder Weg 1b, D-38124 Braunschweig (DSMZ) am 20. Juni 2005 unter der Hinterlegungsnummer DSM 17406 hinterlegt.

Der Stamm *Escherichia coli* BL21 *phoB*::*Tn*10 pagp wurde bei der DSMZ am 20. Juni 2005 unter der Hinterlegungsnummer DSM 17407 hinterlegt.

Der Stamm *Escherichia coli* BL21 *phoB*::*Tn*10 pphy45FLAG wurde bei der DSMZ am 20. Juni 2005 unter der Hinterlegungsnummer DSM 17408 hinterlegt.

Der Stamm *Escherichia coli* BL21 *phoB*::*Tn*10 pphy45STOP wurde bei der DSMZ am 20. Juni 2005 unter der Hinterlegungsnummer DSM 17409 hinterlegt.

Der Stamm *Escherichia coli* BL21 *phoB*::*Tn*10 pKilappA wurde bei der DSMZ am 20. Juni 2005 unter der Hinterlegungsnummer DSM 17410 hinterlegt.

Der Stamm *Escherichia coli BL21 phoB*::*Tn*10 pKilagp wurde bei der DSMZ am 20. Juni 2005 unter der Hinterlegungsnummer DSM 17411 hinterlegt.

Der Stamm *Escherichia coli* BL21 *phoB*::*Tn*10 pKilphy45FLAG wurde bei der DSMZ am 20. Juni 2005 unter der Hinterlegungsnummer DSM 17412 hinterlegt.

Der Stamm *Escherichia coli* BL21 *phoB*::*Tn*10 pKilphy45STOP wurde bei der DSMZ am 20. Juni 2005 unter der Hinterlegungsnummer DSM 17413 hinterlegt.

**Tabelle 2**

| | |
|---|---|
| ES568 pMCS | ES568 *phoB*::Tn10 pMCS |
| ES568 pkil | ES568 *phoB*::Tn10 pkil |
| ES568 pappA | ES568 *phoB*::Tn10 pappA |
| ES568 pkilappA | ES568 *phoB*::Tn10 pkilappA |
| ES568 pagp | ES568 *phoB*::Tn10 pagp |
| ES568 pkilagp | ES568 *phoB*::Tn10 pkilagp |
| | |
| BL21 | BL21 *phoB*::Tn10 |
| BL21 pMCS | BL21 *phoB*::Tn10 pMCS |
| BL21 pkil | BL21 *phoB*::Tn10 pkil |
| BL21 pappA | BL21 *phoB*::Tn10 pappA |
| BL21 pkilappA | BL21 *phoB*::Tn10 pkilappA |
| BL21 pagp | BL21 *phoB*::Tn10 pagp |
| BL21 pkilagp | BL21 *phoB*::Tn10 pkilagp |
| | BL21 *phoB*::Tn10 pphy45STOP |
| | BL21 *phoB*:Tn10 pphy45FLAG |
| | BL21 *phoB*:Tn10 pkilphy45STOP |
| | BL21 phoB::Tn10 pkilphy45FLAG |
| | |
| MG1655 | MG1655 *phoB*::Tn10 |
| MG1655 pMCS | MG1655 *phoB*::Tn10 pMCS |
| MG1655 pkil | MG1655 *phoB*::Tn10 pkil |
| MG1655 pappA | MG1655 *phoB*::Tn10 pappA |
| MG1655 pkilappA | MG1655 *phoB*::Tn10 pkilappA |
| MG1655 pagp | MG1655 *phoB*::Tn10 pagp |
| MG1655 pkilagp | MG1655 *phoB*::Tn10 pkilagp |
| | MG1655*phoB*::Tn10 pphy45STOP |
| | MG1655*phoB*::Tn10 pphy45FLAG |
| | MG1655*phoB*::Tn10 pkilphy45STOP |
| | MG1655*phoB*::Tn10 pkilphy45FLAG |
| | |
| ES1578 | ES1578 *phoB*::Tn10 |
| ES1578 pMCS | ES1578 *phoB*::Tn10 pMCS |
| ES1578 pkil | ES1578 *phoB*::Tn10 pkil |
| ES1578 pappA | ES1578 *phoB*::Tn10 pappA |
| ES1578 pkilappA | ES1578 *phoB*::Tn10 pkilappA |
| ES1578 pagp | ES1578 *phoB*::Tn10 pagp |
| ES1578 pkilagp | ES1578 *phoB*::Tn10 pkilagp |
| | |
| TG1 | TG1 *phoB*:Tn10 |
| TG1 pMCS | TG1 *phoB*::Tn10 pMCS |
| TG1 pkil | TG1 *phoB*::Tn10 pkil |
| TG1 pappA | TG1 *phoB*::Tn10 pappA |
| TG1 pkilappA | TG1 *phoB*::Tn10 pkilappA |
| TG1 pagp | TG1 *phoB*::Tn10 pagp |
| TG1 pkilagp | TG1 *phoB*::Tn10 pkilagp |
| | TG1 *phoB*::Tn10 pphy45STOP |
| | TG1 *phoB*::Tn10 pphy45FLAG |
| | TG1 *phoB*::Tn10 pkilphy45STOP |
| | TG1 *phoB*::Tn10 pkilphy45FLAG |
| | |
| MG1655 mutL ::Kn | MG1655 mutL ::Kn *phoB*::Tn10 |
| MG1655 mutL ::Kn pMCS | MG1655 mutL ::Kn *phoB*::Tn10 pMCS |
| MG1655 mutL ::Kn pkil | MG1655 mutL ::Kn *phoB*::Tn10 pkil |
| MG1655 mutL ::Kn pappA | MG1655 mutL ::Kn *phoB*::Tn10 pappA |
| MG1655 mutL ::Kn pkilappA | MG1655 mutL ::Kn *phoB*::Tn10 pkilappA |
| MG1655 mutL ::Kn pagp | MG1655 mutL ::Kn *phoB*::Tn10 pagp |
| MG1655 mutL ::Kn pkilagp | MG1655 mutL ::Kn *phoB*::Tn10 pkilagp |
| DH5α (phoA-) | |
| DH5α pMCS | |
| DH5α pkil | |
| DH5α pappA | |
| DH5α pkilappA | |
| DH5α pagp | |
| DH5α pkilagp | |

### Beispiel 10: Selektion von Phytase-Aktivität

### a) Wachstumsbedingungen

Die in Tabelle 2 (siehe Beispiel 9) aufgelisteten Bakterienstämme wurden auf Agarplatten verschiedener Minimalmedien ausgestrichen. Die Medienzusammensetzungen basieren auf MOPS-Puffer (Tabelle 4). MOPS-Puffer wurde hergestellt nach, Neidhardt, Bloch, and Smith, 1974, Culture medium for enterobacteria (J. Bacteriol. 119 :736-47). Den Medienzusammensetzungen wurden generell 22 mmol/l Glucose und 0,3 mmol/l Thiamin zugefügt. Weiter wurden so genannte Micronutrients in der in Tabelle 5 angegebenen Konzentration zugefügt. Je nach Zusammensetzung des Minimalmediums waren anorganisches Phosphat (K₂HPO₄; Piᵢ) und/oder Phytat (Na₁₂C₆P₆O₂₄) enthalten (Tabelle 3).

**Tabelle 3**

| **Medium [mmol/l]** | | **K₂HPO₄** | **Na₁₂C₆P₆O₂₄** |
|---|---|---|---|
| **A** | MOPS | 1,32 | - |
| **B** | MOPS Pᵢ⁻ limitiert | 0,066 | - |
| **C** | MOPS Phytat | - | 1,32 |
| **D** | MOPS Phytat-limitiert | - | 0,246 |
| **E** | MOPS Phytat, Pᵢ-limitiert | 0,066 | 1,32 |
| **F** | MOPS Phytat, Pᵢ-hyper-limitiert | 0,0132 | 1,32 |
| **G** | MOPS Phytat-limitiert, Pᵢ-limitiert | 0,066 | 0,246 |
| **H** | MOPS Phytat-limitiert, Pᵢ-hyper-limitiert | 0,0132 | 0,246 |

**Tabelle 4**

| **MOPS-Puffer [mmol/l]** | |
|---|---|
| NH₄Cl | 9,52 |
| M₉Cl₂ | 0,532 |
| K₂SO₄ | 0,276 |
| FeSO₄ · H₂O | 0,010 |
| CaCl₂ · H₂O | 5 x 10⁻⁴ |
| NaCl | 50 |
| MOPS | 40 |
| Tricine | 4 |
| Micronutrients | + (siehe Tabelle 5) |

**Tabelle 5**

| **Micronutrients [mmol/l]** | |
|---|---|
| (NH₄)₆(MO₇)₂₄ | 3 x 10⁻⁶ |
| H₃BO₃ | 4 x 10⁻⁴ |
| CoCl₂ | 3 x 10⁻⁵ |
| CuSO₄ | 10⁻⁵ |
| MnCl₂ | 8 x 10⁻⁵ |
| ZnSO₄ | 10⁻⁵ |

Die Wachstumsstudien konnten bei Temperaturen von 25 °C bis 37 °C durchgeführt werden. Zweckmäßigerweise wurden die Agarplatten über 7 Tage bei 25 °C beziehungsweise 30 °C inkubiert. Das Zellwachstum wurde täglich überprüft.

### b) Ergebnisse

### Verfügbarkeit der Ergebnisse

Eindeutige Ergebnisse wurden bereits nach 3 beziehungsweise maximal 4 Tagen, je nach verwendetem Stamm, erhalten. Eine weitere Inokulation würde sich demnach erübrigen.

### Expression von AppA in E. coli

Es zeigt sich, dass *E.coli phoB*-Stämme, die das Plasmid-kodierte AppA-Protein (Phytase I) oder Glucose-1-Phosphatase (Phytase II) exprimierten, auf Minimalmedien (Medium D), die Phytat als einzige Phosphatquelle enthielten, wachsen konnten. Die Selektion auf Phytaseaktivität konnte daneben mit weiteren Phytat-limitierten Medien mit von 0,1 bis 0,4 mmol/l Phytat durchgeführt werden. Idealerweise betrug die Phytatkonzentration von 0,15 bis 0,4 mmol/l.

### Expression von PhyA aus Bacillus amyloliquefaciens FZB45

*E. coli phoB*-Stämme (BL21 *phoB*::Tn10), die das Plasmid-kodierte PhyA-Protein aus *Bacillus amyloliquefaciens FZB45)* exprimierten, konnten ebenfalls auf Minimalmedien (Medium D), die Phytat als einzige Phosphatquelle enthielten, wachsen (siehe Figuren 3A, 3B, 3C, 3D). Die Selektion auf Phytaseaktivität konnte daneben mit weiteren Phytat-limitierten Medien mit von 0,1 bis 0,4 mmol/l Phytat durchgeführt werden. Idealerweise betrug die Phytatkonzentration von 0,15 bis 0,4 mmol/l.

### Kontrolle

Unter den gleichen Bedingungen (Medium D) konnte im Vergleich dazu bei untransformierten Wirtszellen beziehungsweise bei Wirtszellen, die mit den Plasmiden pMCS oder pkil transformiert wurden, keinerlei Wachstum festgestellt werden (siehe Figuren 3A, 3B, 3C, 3D); diese Wirtszellen exprimieren allein das natürlich vorkommende chromosomal kodierte AppA-Protein.

### Quantifizierung der Aktivität

Weiter konnte festgestellt werden, dass die beobachtete Wachstumsrate unmittelbar mit der Aktivität der exprimierten Phosphatase korreliert; dies konnte durch die parallel durchgeführten quantitativen Western-Plot-Analysen zur Quantifizierung der Expressionsintensität und durch quantitative Experimente in der Flüssigphase zur Bestimmung der Enzymaktivität verifiziert werden.

### Übertragbarkeit

Diese Ergebnisse konnten auch mit den weiteren hergestellten Stämmen (Tabelle 2) beziehungsweise den weiteren Expressionsvektoren bestätigt werden.

## Patentansprüche

1. Verfahren zur Selektion eines Nukleinsäuremoleküls kodierend für eine ausgewählte Phosphatase-Aktivität aus einer Gruppe zu untersuchender Nukleinsäuremoleküle, enthaltend die Schritte:
a) Bereitstellen des zu untersuchenden Nukleinsäuremoleküls,
b) Einführen des zu untersuchenden Nukleinsäuremoleküls und eines Nucleinsäuremoleküls kodierend für das Kil-Protein des Phagen Lambda in eine Phosphatase-defiziente Wirtszelle,
c) Kultivieren der Wirtszelle in Mangelmedium für anorganisches Phosphat, welches das Substrat der ausgewählten Phosphatase-Aktivität als einzige Phosphatquelle und in ausreichender Menge enthält, und
d) Nachweisen des Wachstums der Wirtszellen in dem Medium, wobei Wachstum die Gegenwart des Nukleinsäuremoleküls kodierend die ausgewählte Phosphatase-Aktivität anzeigt.

2. Verfahren nach Anspruch 1, wobei Schritt b) folgende Unterschritte enthält:
b1) Klonieren des Nukleinsäuremoleküls in einen Expressionsvektor,
b2) Transformieren der Wirtszelle mit dem Expressionsvektor.

3. Verfahren nach Anspruch 1 oder 2, wobei das Nukleinsäuremolekül kodierend für das Kil-Protein des Phagen Lambda eine Länge von 287 bp hat und erhältlich ist durch die PCR-Amplifikation genomischer DNA des Phagen Lambda mit dem Primerpaar mit den SEQ ID NO: 1 und SEQ ID NO: 2.

4. Verfahren nach einem der vorstehenden Ansprüche, wobei die Phosphatase-defiziente Wirtszelle eine *E. coli* Zelle ist.

5. Verfahren nach Anspruch 4, wobei die Wirtszelle ein *phoB-*Stamm von *E. coli* ist.

6. Verfahren nach einem der vorstehenden Ansprüche, wobei das Mangelmedium, das Substrat, in einer Konzentration von 0,1 bis 0,5 mmol/l enthält.

7. Verfahren nach einem der vorstehenden Ansprüche, wobei die Wirtszellen in dem Mangelmedium von 2 bis 4 Tage kultiviert werden.

8. Verfahren nach einem der vorstehenden Ansprüche, wobei die Wirtszellen in dem Mangelmedium von 28 bis 32 °C kultiviert werden.

9. Verfahren nach einem der vorstehenden Ansprüche, wobei die ausgewählte Phosphatase die Phosphatase eines gram-negativen Bakteriums ist.

10. Verfahren nach einem der vorstehenden Ansprüche, wobei die ausgewählte Phosphatase eine intrazelluläre Phosphatase ist.

11. Verfahren nach Anspruch 10, wobei die Phosphatase eine periplasmatische Phosphatase ist.

12. Verfahren nach einem der vorstehenden Ansprüche, wobei die ausgewählte Phosphatase eine Phytase ist und das Substrat der ausgewählten Phosphatase Phytinsäure und/oder Phytat ist.

13. Verwendung eines Nukleinsäuremoleküls kodierend für das Kil-Protein des Phagen Lambda zur Selektion von Nukleinsäuremolekülen kodierend für Phosphatase-Aktivität.

14. Verwendung nach Anspruch 13, wobei das Verfahren nach einem der Ansprüche 1 bis 12 durchgeführt wird.

## Claims

1. Method for selection of a nucleic acid molecule coding for a selected phosphatase activity from a group of nucleic acid molecules to be analyzed, comprising the steps:
a) providing the nucleic acid molecule to be analyzed,
b) inserting the nucleic acid molecule to be analyzed and a nucleic acid molecule coding for the Kil protein of phage lambda into a phosphatase deficient host cell,
c) culturing the host cell in deficiency medium for inorganic phosphate containing the substrate of the selected phosphatase activity as the sole phosphate source and in sufficient amount and
d) detecting the growth of the host cells in the medium, wherein growth indicates the presence of the nucleic acid molecule coding for the selected phosphatase activity.

2. Method according to claim 1, wherein step b) comprises the following substeps:
b1) cloning the nucleic acid molecule in an expression vector,
b2) transforming the host cell with the expression vector.

3. Method according to claim 1 or 2, wherein the nucleic acid molecule coding for the Kil protein of phage lambda has a length of 287 bp and is accessible by PCR amplification of genomic DNA of phage lambda with the primer pair having SEQ ID NO: 1 and SEQ ID NO: 2.

4. Method according to any one of the preceding claims, wherein the phosphatase-deficient host cell is an *E*. *coli* cell.

5. Method according to claim 4, wherein the host cell is a *phoB* strain of *E*. *coli.*

6. Method according to any one of the preceding claims, wherein the deficiency medium contains the substrate in a concentration of 0.1 to 0.5 mmol/L.

7. Method according to any one of the preceding claims, wherein the host cell is cultured in the deficiency medium for 2 to 4 days.

8. Method according to any one of the preceding claims, wherein the host cells are cultured in the deficiency medium at 28 to 32°C.

9. Method according to any one of the preceding claims, wherein the selected phosphatase is the phosphatase of a gram-negative bacterium.

10. Method according to any one of the preceding claims, wherein the selected phosphatase is an intracellular phosphatase.

11. Method according to claim 10, wherein the phosphatase is a periplasmic phosphatase.

12. Method according to any one of the preceding claims, wherein the selected phosphatase is a phytase and the substrate of the selected phosphatase is phytic acid and/or phytate.

13. Use of a nucleic acid molecule coding for the Kil protein of phage lambda for selection of nucleic acid molecules coding for phosphatase activity.

14. Use according to claim 13, wherein the method is carried out according to any one of the claims 1 to 12.

## Revendications

1. Procédé de sélection d'une molécule d'acide nucléique codant pour une activité de phosphatase sélectionnée parmi un groupe de molécules d'acide nucléique à examiner, contenant les étapes de :
a) préparation de la molécule d'acide nucléique à examiner,
b) introduction de la molécule d'acide nucléique à examiner et d'une molécule d'acide nucléique codant pour la protéine Kil du phage lambda dans une cellule hôte déficiente en phosphatase,
c) culture de la cellule hôte dans un milieu déficient en nutriments pour le phosphate inorganique qui contient le substrat de l'activité de phosphatase sélectionnée comme source de phosphate unique et en quantité suffisante, et
d) preuve de la croissance des cellules hôtes dans le milieu, la croissance indiquant la présence de la molécule d'acide nucléique codant pour l'activité de phosphatase sélectionnée.

2. Procédé selon la revendication 1, dans lequel l'étape b) contient les étapes secondaires suivantes :
b1) clonage de la molécule d'acide nucléique dans un vecteur d'expression,
b2) transformation de la cellule hôte avec le vecteur d'expression.

3. Procédé selon la revendication 1 ou 2, dans lequel la molécule d'acide nucléique codant pour la protéine Kil du phage lambda a une longueur de 287 pb et peut être obtenue par l'amplification par PCR d'ADN génomique du phage lambda avec la paire d'amorces avec les SEQ ID N° : 1 et SEQ ID N° : 2.

4. Procédé selon l'une quelconque des revendications précédentes, dans lequel la cellule hôte déficiente en phosphatase est une cellule de *E. coli.*

5. Procédé selon la revendication 4, dans lequel la cellule hôte est une souche *phoB* de *E. coli.*

6. Procédé selon l'une quelconque des revendications précédentes, dans lequel le milieu déficient en nutriments contient le substrat dans une concentration de 0,1 à 0,5 mmol/l.

7. Procédé selon l'une quelconque des revendications précédentes, dans lequel les cellules hôtes dans le milieu déficient en nutriments sont cultivées pendant 2 à 4 jours.

8. Procédé selon l'une quelconque des revendications précédentes, dans lequel les cellules hôtes dans le milieu déficient en nutriments sont cultivées à 28 à 32°C.

9. Procédé selon l'une quelconque des revendications précédentes, dans lequel la phosphatase sélectionnée est la phosphatase d'une bactérie Gram-négative.

10. Procédé selon l'une quelconque des revendications précédentes, dans lequel la phosphatase sélectionnée est une phosphatase intracellulaire.

11. Procédé selon la revendication 10, dans lequel la phosphatase est une phosphatase périplasmatique.

12. Procédé selon l'une quelconque des revendications précédentes, dans lequel la phosphatase sélectionnée est une phytase et le substrat de la phosphatase sélectionnée est l'acide phytique et/ou le phytate.

13. Utilisation d'une molécule d'acide nucléique codant pour la protéine Kil du phage lambda pour la sélection de molécules d'acide nucléique codant pour l'activité de phosphatase.

14. Utilisation selon la revendication 13, dans laquelle le procédé selon l'une quelconque des revendications 1 à 12 est mis en oeuvre.
